# EUROPEAN PATENT SPECIFICATION

(11) **EP 2 873 364 B1**
(45) Date of publication and mention of the grant of the patent: **08.03.2017**
(21) Application number: 13193137.0
(22) Date of filing: 15.11.2013
(51) Int. Cl.: A61B 3/06

(54) **Multispectrally tested, printed colour vision test for the fine evaluation of the degree of deficiency**
Multispektral getesteter Druckfarbensehtest zur feinen Auswertung des Mangelgrades
Test de vision de couleur imprimé, testé de manière multi spectrale pour l'évaluation précise du niveau de déficience

(43) Date of publication of application: 20.05.2015
(73) Proprietor: Institute of Solid State Physics, University of Latvia, 1063 Riga (LV)
(72) Inventor: Fomins, Sergejs, LV-1019 Riga (LV); Ozolins, Maris, LV-1007 Riga (LV)
(74) Representative: Kuzjukevica, Lucija

(56) References cited:
- FR-A1- 2 722 081
- Shinobu Ishihara: "Test for color blindness" In: "Test for color blindness", 1 January 1972 (1972-01-01), Tokyo, Kyoto, XP055116830, * tables 1,16 *

## Description

### Technical Field

The invention relates to tools and methods of eye examining, and more specifically to colour vision tests, including colour deficiency or colour sensitivity tests.

### Background Art

Most frequently used colour vision tests are pseudo-isochromatic (PIH) plates. A typical test would consist of an object on the background made up of many elements (typically of circles or other shapes) [Hardy]. The differently shaded and sized elements would fill the entire area of the test. A test symbol would be formed by colouring the respective elements. The colour tone determines the possibility of diagnosing a type of colour vision deficiency. The difference in colour saturation, on the other hand, is used for quantitative diagnostics. Polychromatic plate tests are used for relatively quick detection of the red-green and blue-yellow deficiency. Some tests are designed for the diagnostics of the extent of deficiency (Ishihara, Rabkin, HRR). Currently available PIH tests diagnose three levels of the red-green deficiency: weak, medium and strong (*Richmond HRR, 4th Ed*.)*.* The tests are very different in terms of selected type and number of the hidden objects as well as interpretation of answers [Cole, 2007].

It is known that there are more than three types of variation in the human visual pigment [Asenjo et al, 1994]. In different circumstances changes in macular pigment optical density may occur. The two subtypes of long wavelength sensitive cones in males are at 555 and 559nm. [Neitz, 1986; Neitz, 2011] Genetic changes in LWS and MWS pigments as well as in the optical density result in more than three deficiency levels.

Due to the fact that the tests come in a physical form, i.e., are printed, they have a number of negative characteristics that influence their performance. Firstly, the selected lighting and the features of spectral reflectance of printing technology affect the test results and may lead to misdiagnosis or complete loss of test performance. Secondly, the pages of the printed tests become yellowed over time and thirdly, printing pigments lose colour or fade. Fourthly, high quality tests are produced using sophisticated printing technologies, which in turn increases the costs of production.

FR 2 722 081 A discloses a test for colour blindness comprising test symbols on a background of square elements.

Human vision adapts to the environment when the rearrangement of signals in the eye's photoreceptors and further in neural pathways takes place. Coloured objects are being separated from the background. Thus, by performing a colour vision test using only one type of the plates, there is a risk of making a superficial assessment of colour vision. This is the basis of the major differences in the assessment results when using different tests.

What is needed is an accessible print for colour vision test adapted to be assessed in the available commercial lightings. It is necessary to establish the testing feasibility in different settings, in any indoor lighting conditions. Optimal colorimetric properties must be achieved. The test must be suitable for determining more deficiency severity levels (e.g., five) compared to existing tests. Existing tests do not allow applying the psychometric mathematics. In order to use the mathematical apparatus, it is necessary to increase the amount of information. Number of test symbols could be increased and diagnostic value could be improved, while keeping the number of test pages the same, which will help to maintain the same testing time.

### Summary of invention

The test comprises plates made in ink on paper or similar substrate. The test comprises three types of plates, which can be distinguished by the background properties. The first type of plates comprises plates with the same-size square test symbols on a same-size square background. The second type of plates comprises plates with the test symbols on a grey circle background. The third type of plates comprises plates with the test symbols on a coloured circle background. All of the test symbols comprise elements that create background.

The background elements and symbols are preferably randomly generated using a mathematical algorithm. While forming the elements into symbols placement of elements, their size and brightness could be varied to maintain optimal appearance of symbol on background. The elements preferably have three discrete levels of brightness: bright, medium and dark. Accordingly there are also three levels of brightness for the coloured test symbols. As the symbols for circle based plates (i.e., of first and second type as described above) were selected easily recognizable geometrical shapes, such as circle, square, rhombus and cross. Preferably, the sequence of shapes in these test plates is chosen so that it never recurs.

For the square based plates (i.e., of first type as described above), Arabic numerals from 0 to two-digit numbers may be used. The sequence (combination) of the numbers does not recur in any of the plates.

For the second and third type of the plates each plate preferably contains four test symbols. For the third type of the plates each plate preferably contains three test symbols.

The test preferably contains 17 plates, 15 of which are adapted for the determination of the red-green vision deficiencies whereas the other two plates - for the determination of the blue-yellow type of vision deficiency. The first 15 plates, depending on the colour saturation, are preferably divided into five categories. Each category contains three plates of every type described above. These categories are designed to determine the severity of the colour blindness, i.e., the first category is designed for the determination of dichromacy and the fifth category is designed for determining minimal deficiency. Accordingly, where the category increases, the colour saturation decreases. Thus, during the examination, a tested person goes through the different levels of difficulty - from easily visible plates (high saturation) to those hard to see (low saturation).

The blue-yellow plates are formed of grey circles in three different levels of brightness. The test symbols are geometric shapes. The test plate contains four symbols, each with its own colour saturation. The symbols of the plate No 16 are blue, whereas the symbols of the plate No17 - yellow. The colours correspond to the theoretical confusion lines in case of tritanopic colour vision deficiency.

A colour of symbols had been selected based on the dichromatic confusion lines [Pitt; Stockman]. In order to ensure colour consistency, the selected colour printer was spectrally calibrated. Based on the *Neugebauer* approach [Raja Balasubramanian, 1995], the mathematical apparatus for colour description, including *Demichelis* equations [Raja Balasubramanian 2003, Green] and using the CMYK technology for colour coding, was set up.

The inspection of the printed tests is carried out in two stages by evaluating the consistency of the absolute spectral reflections with the confusion lines and by using multispectral imaging. The performance of the created plates was analysed in natural and artificial lighting conditions. For this purpose, a multispectral photo of a physical test in the appropriate lighting settings is taken, using a calibrated multispectral camera. The created algorithms are applied to the spectral image and the common physiological response evoked by the test and the lighting settings is analysed in the vision analyser.

### Brief description of drawings

Fig. 1 A shows the first type plates, each plate consisting of the same-size squares as well as of numerals as the test symbols;
Fig. 1 B shows the second type plates, each plate consisting of the circles of four different sizes as well as of geometric shapes as the test symbols;
Fig. 1 C shows that the third type plates, each plates having a background comprising coloured circles of various sizes and different brightness; the test symbols are geometric shapes.
Fig. 2 is a schematic layout of the test plates and consistency with the degree of strength.

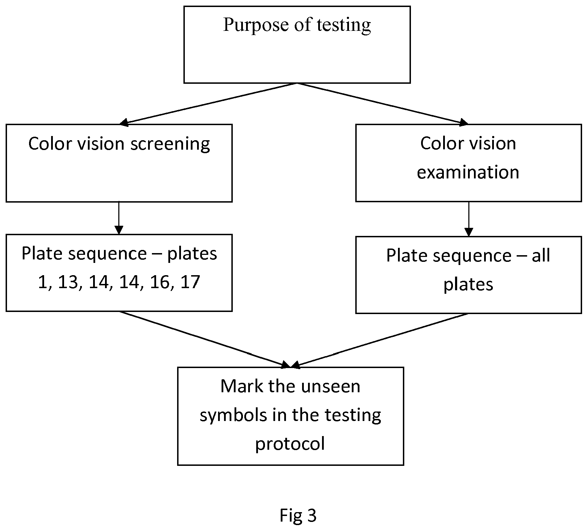

Fig. 3 shows the testing flowchart.
Fig. 4 describes the tuning-up of the test plates' analysis and the CMYK colour recipe, using multispectral analysis.
Fig 5 is a chart showing common ratio of the seen symbols in five categories according to the test.
Fig 6 is a chart showing correlation between the unseen numerals in the developed test and the unseen symbols in the HRR 4th Ed. test.
Fig 7 shows an exemplary test results of a real observer.
Fig 8 shows a chart of ratio of recognised symbols according to real test results and sigmoids calculated from said test results for protan and deutan digits.

### Description of embodiments

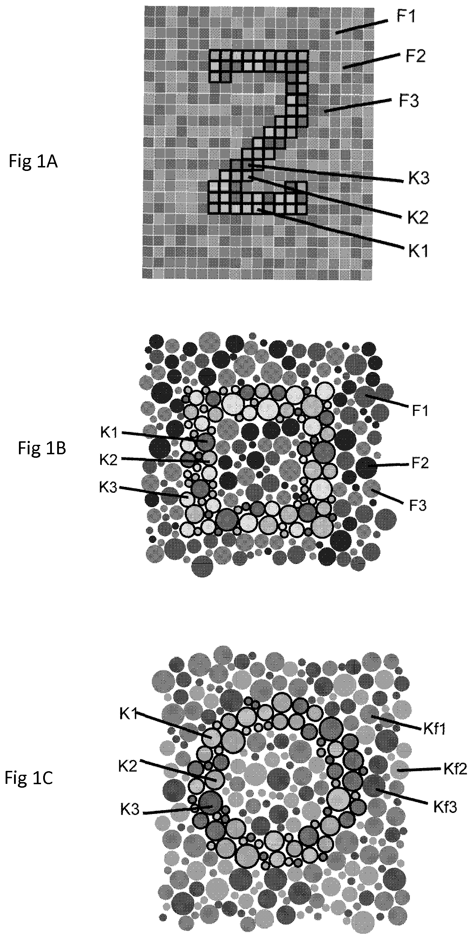

One embodiment of the invention is now described in reference to Figs 1A to 1C. The Figs in this patent application are presented in grayscale, but the plates in fact are in colour. The elements making up the test symbols are shown with surrounding line; such line is just to indicate such test elements and are not present in actual plates. The colour of the background elements in the first type plate is preferably grey - indicated as F1, F2, F3 on Fig.1 A. The size of the elements forming both the background and the test symbols is the same. The elements forming the symbols differ in colour from those symbols forming the background. The stimulus colours (K1, K2, K3) have been chosen, based on the confusion lines of the CIE xy or CIE uv chromaticity diagram. The brightness of the colours K1, K2, K3 shifts accordingly to the brightness of the grey background colours F1, F2, F3. The grey colour within the given plates serve as a point of reference for calculating colour saturation of the symbols in the colour space. High colour saturation allows identifying the absolute or strong colour vision deficiencies, whereas the low colour saturation is for determining minor colour vision deficiencies.

The identical situation is shown in Fig.1 B, where the choice of element colour is based on similar principles. The symbol colour in the third type plates in Fig.1 C is identical to that of the second type plates in Fig.1 B, but the grey background colour is replaced by the red symbol colour, making the red stimulus colour invisible and thus the plate has three symbols. These type of plates are more easily viewable by people without colour vision deficiencies due to the higher colour contrast. For people with colour vision deficits spotting the symbols is as difficult as spotting them on the grey background due to the background colour being equivalent to the grey colour from the vision deficiency perspective. Where the colour vision is normal, the second red symbol is hard to spot, but it becomes easily visible in case of colour deficiency. This serves as an additional tool for identifying colour deficiency.

There are four different sizes for the elements forming the plates shown in Fig.1 Band C.

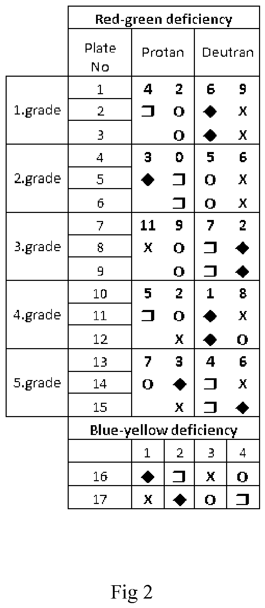

Fig. 2 shows the layout of the test and the symbols in each plate. Each degree of the red-green deficiency corresponds to the three plates of the same colour recipe and accordingly identical colour saturation and colorimetric coordinates. The plates contain symbols for determining Protanopia, Protanomaly, Deuteranopia and Deuteranomaly, as well as Tritanopy. There are 11 symbols in total in each level for detection of red-green colour vision defects. For diagnosis of the Protanopia or Protanomaly and Deuteranopia or Deuteranomaly type of deficiencies five symbols for each deficiency type per each level are used. The use of such a great number of symbols allows for determination of psychophysical thresholds and a more accurate evaluation of the degree of deficiency.

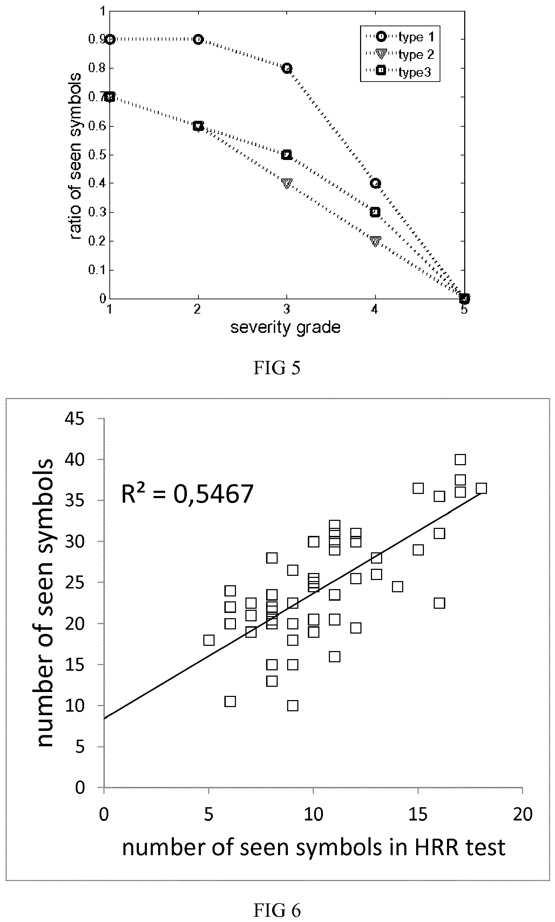

Figs 5 to 8 illustrate the results of a real test. Fig 5 represents a common ratio of the seen symbols in five categories (n=54). The ratio of the seen symbols for each level of test and three types of plates. Line with circles represent recognised numerals on the square background (first type). Line with squares represent recognised shapes on the grey circle background (second type). Line with triangles represent recognised shapes on the coloured circle background (third type).

The numeral plates are easier to spot in case of the equivalent colour saturation.

Fig. 6 illustrates the correlation between the unseen numerals in the developed test and the unseen symbols in the HRR 4th ed. test. The high positive correlation can be observed.

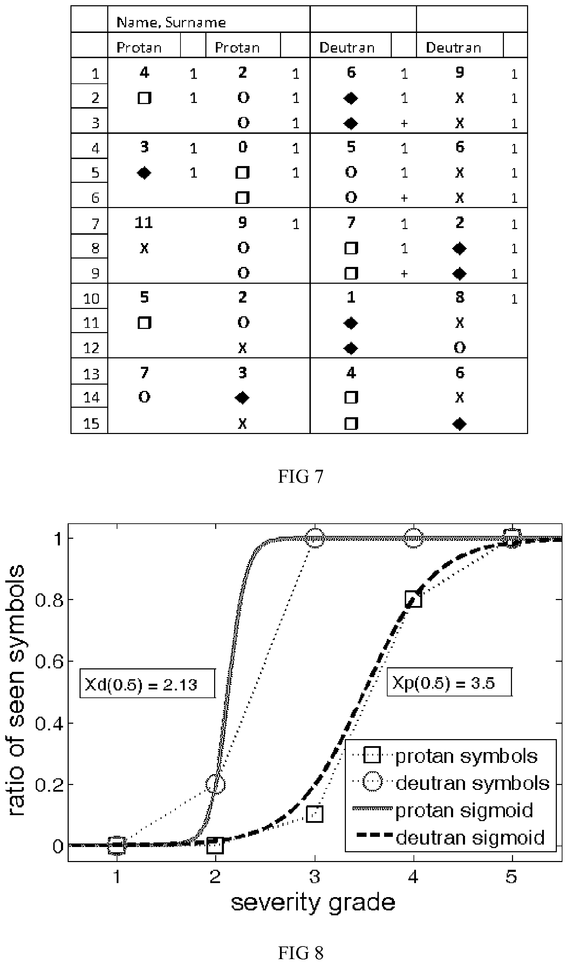

Fig. 7 shows the test results of a real observer. The table provides the exemplary results by a real observer with the total number of symbols seen as 10:16. This means that the subject has the protanomaly, since he is able to better recognize the deuteranopia related symbols. "1" in the table means that a symbol was recognized.

If we count the number of the seen symbols for each category, we obtain:
- protan - 5, 4, 1, 0, 0;
- deutan - 5, 5, 5, 1, 0.

By normalising the results (so that the five becomes 1), we obtain a graphical representation of the data. The protan symbol ratio on Fig 8 is shown as the squares, whereas the deutan symbol ration is shown as circle symbols.

By applying the mathematical description of these curves with the Boltzmann sigmoid function we obtain the x-axis values, which correspond to the midpoint of function (y = 0.5). Midpoint of sigmoid represents the value, when half of the symbols at the particular level are seen. A value below this point indicates that only one or two symbols of five are seen, and is interpreted as failed. Values higher than 0.5 indicate that three of more symbols were seen (pass criteria). For the deutan type symbols midpoint of sigmoid stands at 2.13 and for the protan - 3.5., which is the severity level indicator. In this particular case, the strong degree of protanomaly has been determined.

The colour saturations for all three plates in each category are identical. Considering that the composition of plates is different, the plates can be used to determine various degrees of deficiencies. In comparison to the second and third type of plates the first type of plates are more easily readable, whereas the second and third type of plates in terms of their strength are similar.

The plates designed for the blue-yellow type of deficiencies are set separately in the end of the test. The symbols are tiered in four levels of strength. One plate contains four symbols. In the first blue-yellow type plate the symbols consist of blue tone pigments. The symbols of the second plate are yellowish. The colours had been selected based on the dichromatic confusion lines for the tritanopic colour vision deficiency. The background and symbols consist of circles and correspond to the type of plates shown in Fig.1 B.

The test plates can also have different sequence layout to ensure that person have not recognized the plates. However, the plates for determination of strong deficiencies (number one to three) must always be used in the beginning as an introduction for both people with and without colour vision deficiencies.

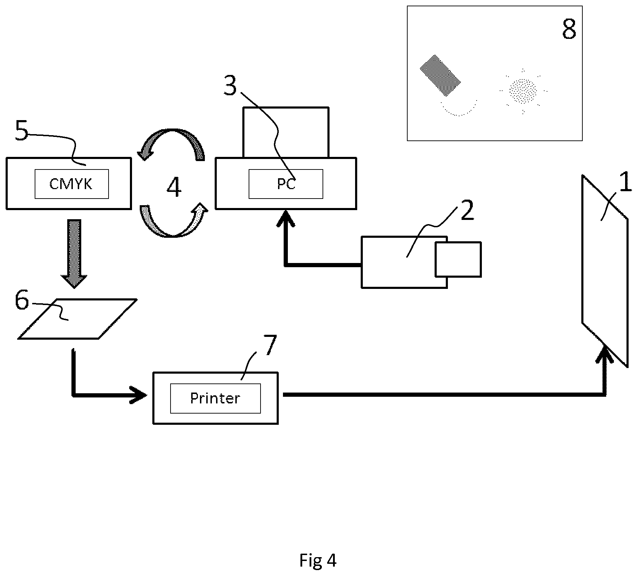

During various phases of the test development several analytical methods were applied. First, the correspondence of the selected colours' spectral reflections to the confusion lines was analysed [Fomins, et al]. Second, in order to improve the colorimetric quality indicators as well as to verify feasibility of the test in indoor lighting conditions, multispectral analysis of the test plates was carried out. Fig.4 shows the analysis scheme allowing spectral calibration of the test colours with feedback. In Fig.4, a colour set or the test plate 1 is recorded by camera 2 in particular lighting settings 8. With the help of computer 3 the colorimetric values/brightness of the colour samples/test plate are analysed and where necessary the changes are made in the colour recipe 5. By implementing the changes in a version of colour samples/test plate printing is performed on the accordingly calibrated printer 7.

Feedback procedure for colour correction have two phases. First, colour receipt is selected through using Demichelis equations and Neugebauer approach based mathematics. Prior to that, ink coverages for graduated values of CMYK are acquired, and look-up table (LUT) is created for particular printer, with particular settings. Further, based on LUT the theoretical CMYK receipt is provided, to account for confusion lines, brightness, and colour saturation. In this first step, initial colour samples 1 (not yet test) are printed and placed for imaging under illumination source 8. Imaging is performed by spectrally calibrated multispectral (MS) camera 2 CRI Nuance VIS07 in the range of 420 to 720 nm visual wavelength range. Spectral images are analysed by a PC 3. Acquired colorimetric data is compared to initial theoretical CMYK receipt 5, corrections 4 are introduced, if needed. Correction are necessary if the brightness do not match or/and chromaticity do not match confusion lines. Mistakes in the colour receipt can be due to mistakes in spectral measurement of CMYK samples, theoretical description of colour do not match physical printing, printer setting changes.

After the feedback a new receipt is provided 5, Digital versions of test plates 6 are filled with colour from new receipt 5 and printed by calibrated printer 7. Printed test plates 1a are then captured by MS camera 2 and analysed by digital algorithm proposed by Fomins et al [Fomins] as follows. Multipectral images were taken with CRI Nuance VIS 07 camera equipped with liquid crystal tunable filters in the range of 420 nm to 720 nm in 10 nm step. Spectral characteristics of the multispectral system were corrected to the measurements made with radiometrically calibrated USB 4000 spectrometer (Ocean Optics) to the narrow band fluorescent lights. Spectral sensitivity was corrected for the full-spectra natural daylight.

The model could be divided into three stages. First, multispectral image is transformed into cone excitation images. For this purpose we use latest Stockman and Sharpe [Stockman] cone fundamentals for two degree observer. Each of the spectral images is multiplied by corresponding cone excitation value at defined wavelength and all spectral images are summed into one composite image. At this stage three images corresponding to data captured at LWS, MWS and SWS cones are obtained. Anomalous trichromacy vision modes and dichromacy are introduced at this stage. The modes of vision are controlled by spectral separation of LWS cones for simulation of protanomaly and MWS cones for simulation of deuteranomaly. Dichromacy is simulated when the spectral sensitivities of LWS and MWS are similar.

After, cone signal wiring mathematics was applied to be used in later analysis. The channels responsible for red-green vision S+(L-M) was chosen for evaluation of test performance. In case of dichromacy the equation becomes S+L or S+M, and for anomalous trichromatic visions S+(L-L'), S+(M'-M) .

The process is dependent on different gains of color receptors, which we suppose also works for dichromatic color vision. In our model L and M cone signals were left constant, while S cone signal gain was automatically selected to account for minimal contrast variations resulting S+(L-M) image. This allowed obtaining maximal contrast between background and latent symbols.

If the results of algorithm are not satisfying, new feedback loop is provided with new corrections to CMYK receipt 5.

Multispectral imaging in combination with the developed algorithm is used for determination of the test feasibility in a variety of lighting settings. According to the described algorithm [Fomins], the plates are assessed in terms of their quality.

The drop-out criteria for the test is greater than that for the HRR test - two errors [Cole, 2006]. For people with colour vision deficiencies the smallest number of errors allowed is six, while for the people with normal trichromatic colour vision - no more than three mistakes are allowed.

The five categories developed ensure a more accurate diagnosis of the vision deficiency character. This allows determining more precisely the vision standards for various professions.

The variety of test types ensures a comprehensive assessment of the vision deficiencies in connection with different types of stimulus. The adjusted result allows for a more objective assessment of the degree of colour deficiency.

The calculated classification of the red-green deficiency (protan - or deutan) for any of the three types of the plates always results in an unequivocal diagnosis. For instance, for the first type - protan, for the second type - deutan and for the third type - deutan, meaning 1:2 or the deutan type deficiency.Such a novel approach helps to determine the type of deficiency even if the total number of symbols viewed for the protan and deutan deficiencies coincide. During the test development phase the multispectral analysis method is applied, ensuring the colorimetric consistency of the dichromatic confusion lines in the selected lighting settings.

In addition, the algorithm [Fomins] that evaluates the diagnostic capacity of the test in different lighting settings was applied.

Compared to other test results, the developed test allows for a more accurate diagnosis of the type of red-green deficiency.

The above mentioned improvements combined with the analytical methods, enhance the differentiation of the type of deficiency within a range of lighting settings.

By using spectrally calibrated printer, a mathematical model is applied and the test target printing is carried out. The target is spectrally analysed using a spectrometer and a multispectral camera. The corrections are made if necessary. The electronic version of the test is supplemented with the correct colour recipes.

Colour vision test can be carried out in the lighting of at least 500 lux. As the light source must be used daylight, excluding any direct rays, or the fluorescent lighting with a colour temperature of 4000 to 6500 K and a colour rendering index greater than 70 units, cold LED lighting. The tested person should be at the distance of 40-50 cm from the test. The test should be placed on the table. It is acceptable to hold the test in hands.

Colour vision screening: begins with a demonstration of the first three cards. Then the test proceeds directly to the fifth block and the testing continues using five discrete cards.

Examination of the red-green vision: begins with the first card as a demonstration card and goes through the remaining set of 15 cards.

Examination of the blue-yellow vision: begins with the first three cards to ensure the understanding of the testing principles and then proceeds with the last two cards.

### Reference signs list

1 - initial colour samples
2 - multispectral camera
3 - computer (PC)
4 - corrections
5 - feedback
6 - digital versions of test plates
7 - calibrated printer
8- illumination source

### Citation list

[Hardy] Le Grand H. Hardy et al, 1960, US Pat.2937567
[Cole, 2007] Cole BL.Assessment of inherited colour vision defects in clinical practice. Clin Exp Optom. 90(3):157-75.
[Pitt] Pitt, F. H. G. (1935). Characteristics of dichromatic vision. Medical Research Council Special Report Series, No. 200. London: His Majesty's Stationery Office.
[Stockman] Stockman, A., & Sharpe, L. T. (2000). Spectral sensitivities of the middle- and long-wavelength sensitive cones derived from measurements in observers of known genotype. Vision Research, 40, 1711-1737.
[Raja Balasubramanian 2003] R. Bala. (2003). Device characterization, Chapter 5, Digital Color Imaging Handbook, Gaurav Sharma Ed., CRC Press.
[Green] Green, P. (2002). Characterizing hard copy printers, in Colour Engineering, P. Green and L.W. MacDonald (Eds). John Wiley & Sons.
[Fomins] Fomins, Ozolin 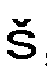 , Luse. (2013) Practical and theoretical evaluation of prined pseudoisochromatic plates for congenital colour visions deficiencies. *ICVS Abstact Book*, p.127.
[Neitz 2011] Neitz, J., Neitz, M. (2011). The genetics of normal and defective color vision. Vision Research 51(7), 633-651.
[Neitz 1986] Neitz, J. & Jacobs, G.H. (1986). Polymorphism of the long-wavelength cone in normal human colour vision. Nature, 323, 623-625
[Asenjo] Asenjo, A.B., Rim, J., Oprian, D.D. (1994). Molecular determinants of human red/green color discrimination. Neuron 12, 1131-1138.
[Raja Balasubramanian 1995] Raja Balasubramanian (1995). A spectral Neugebauer model for dot-on-dot printers, Proc. SPIE vol. 2413.
[Cole, 2006] Cole, BL, Lian KY, Lakkis C (2006). The new Richmond HRR pseudoisochromatic test for colour vision is better than the Ishihara test. Clin Exp Optom 89 (2), 73-80.

## Claims

1. A colour vision deficiency test, comprising a set of test plates, each test plate comprising a background formed from background elements, said set of test plates comprising three types of plates **characterized by** background properties, wherein the first type of plates are plates with test symbols formed from same-size square test symbol elements on a background formed from same-size square background elements, the second type of plates are plates with test symbols on a background formed from grey circle background elements and the third type of plates are plates with test symbols on a background formed from coloured circle background elements.

2. A test as in claim 1, wherein said background elements and symbols are randomly generated.

3. A test as in claims 1 to 2, wherein said elements and said test symbols having three discrete levels of brightness, including bright, medium and dark.

4. A test as in claims 1 to 3, wherein said test symbols on said second and third type of plates are selected from a group of easily recognizable geometrical shapes, comprising circle, square, rhombus and cross.

5. A test as in claims 1 to 4, wherein said test symbols on first type of plates are one or two digit Arabic numerals.

6. A test as in claims 1 to 5, wherein said first and second types of the plates contains four test symbols.

7. A test as in claims 1 to 6, wherein said third type of the plates contains three test symbols.

8. A test as in claims 1 to 7, wherein said test contains 15 plates that are adapted for the determination of red-green vision deficiencies and two plates adapted for the determination of the blue-yellow type of vision deficiency.

9. A test as in claim 8, wherein said first 15 plates for determining red-green type of vision deficiencies are divided into five categories, each category comprising at least one plate of every type, wherein said five categories are designed to determine minimal, mild, medium, strong, and dichromatic colour vision deficiency correspondingly.

10. A test as in claim 8, wherein said two plates for determining blue-yellow type of vision deficiencies are formed of grey circles in three different levels of brightness and wherein each test plate contains four test symbols each with its own colour saturation, wherein the test symbols are geometric shapes.

11. A test as in claim 10, wherein one of said test plates comprise test symbols of blue tone.

12. A test as in claim 10 to 11, wherein one of said test plates comprises test symbols of yellow tone.

13. A method for preparing a colour vision deficiency test according to claims 1 to 12, said method comprising selecting a colour receipt, acquiring ink coverages for graduated values of CMYK, creating a look-up table for a particular printer with particular settings, providing a theoretical CMYK receipt based on said look up table , to account for confusion lines, brightness, and colour saturation, printing initial colour samples, analyzing said initial colour samples under actual colour conditions; imaging said initial colour samples using calibrated multispectral camera, comparing said acquired colorimetric data with theoretical CMYK receipt, and printing test plates using corrected CMYK receipt.

## Patentansprüche

1. Farbenfehlsichtigkeitstest, umfassend einen Satz Testplatten, wobei jede Testplatte einen aus Hintergrundelementen gebildeten Hintergrund umfasst, wobei der Satz Testplatten drei Typen von Platten umfasst, die charakterisiert sind durch Hintergrundeigenschaften, wobei der erste Typ von Platten Platten sind mit Testsymbolen, die aus gleich großen Quadrat-Testsymbolelementen auf einem Hintergrund gebildet sind, der aus gleich großen Quadrat-Hintergrundelementen gebildet ist, wobei der zweite Typ von Platten Platten sind mit Testsymbolen auf einem Hintergrund, der aus grauen Kreis-Hintergrundelementen gebildet ist und wobei der dritte Typ von Platten Platten sind mit Testsymbolen auf einem Hintergrund, der aus farbigen Kreis-Hintergrundelementen gebildet ist.

2. Test nach Anspruch 1, wobei die Hintergrundelemente und Symbole zufällig generiert werden.

3. Test nach den Ansprüchen 1 oder 2, wobei die Elemente und die Testsymbole drei diskrete Helligkeitsniveaus aufweisen, einschließlich hell, mittel und dunkel.

4. Test nach den Ansprüchen 1 bis 3, wobei die Testsymbole auf den Platten des zweiten und dritten Typs ausgewählt sind aus einer Gruppe von einfach erkennbaren geometrischen Formen, umfassend Kreis, Quadrat, Raute und Kreuz.

5. Test nach den Ansprüchen 1 bis 4, wobei die Testsymbole auf Platten des ersten Typs ein- oder zweistellige arabische Ziffern sind.

6. Test nach den Ansprüchen 1 bis 5, wobei die Platten des ersten oder zweiten Typs vier Testsymbole enthalten.

7. Test nach den Ansprüchen 1 bis 6, wobei die Platten des dritten Typs drei Testsymbole enthalten.

8. Test nach den Ansprüchen 1 bis 7, wobei der Test 15 Platten, die für die Bestimmung von rot-grün Fehlsichtigkeiten angepasst sind, und zwei Platten, die für die Bestimmung der Fehlsichtigkeit des blau-gelben Typs angepasst sind, enthält.

9. Test nach Anspruch 8, wobei die ersten 15 Platten zum Bestimmen von Fehlsichtigkeiten des rot-grünen Typs in fünf Kategorien unterteilt sind, wobei jede Kategorie zumindest eine Platte eines jeden Typs umfasst, wobei die fünf Kategorien ausgestaltet sind, minimale, leichte, mittlere, starke und dichromatische Farbensehschwäche entsprechend zu bestimmen.

10. Test nach Anspruch 8, wobei die zwei Platten zum Bestimmen von Fehlsichtigkeiten des blau-gelben Typs aus grauen Kreisen in drei unterschiedlichen Helligkeitsniveaus gebildet sind und wobei jede Testplatte vier Testsymbole mit jeweils seiner eigener Farbsättigung enthält, wobei die Testsymbole geometrische Formen sind.

11. Test nach Anspruch 10, wobei eine der Testplatten Testsymbole blauer Tönung umfassen.

12. Test nach den Ansprüchen 10 bis 11, wobei eine der Testplatten Testsymbole gelber Tönung umfassen.

13. Verfahren zur Herstellung eines Farbenfehlsichtigkeitstests gemäß den Ansprüchen 1 bis 12, wobei das Verfahren umfasst Auswählen eines Farbrezepts, Erlangen von Farbadeckungen für graduierte Werte von CMYK, Erstellen einer Nachschlagtabelle für einen speziellen Drucker mit speziellen Einstellungen, Bereitstellen eines auf der Nachschlagtabelle basierenden theoretischen CMKY Rezepts, um Verwirrungslinien, Helligkeit und Farbsättigung zu berücksichtigen, Drucken initialer Farbproben, Analysieren der initialen Farbproben unter tatsächlichen Farbbedingungen; Abbilden der initialen Farbproben unter Verwendung einer kalibrierten multispektralen Kamera, Vergleichen der erlangten kolorimetrischen Daten mit dem theoretischen CMYK Rezept und Drucken der Testplatten unter Verwendung des korrigierten CMYK Rezepts.

## Revendications

1. Test de déficience de la vision des couleurs, comprenant un ensemble de plaques de test, chaque plaque de test comprenant un arrière-plan formé à partir d'éléments d'arrière-plan, ledit ensemble de plaques de test comprenant trois types de plaques **caractérisés par** des propriétés d'arrière-plan, dans lequel le premier type de plaques consiste en des plaques avec des symboles de test formés à partir d'éléments de symboles de test carrés de même taille sur un arrière-plan formé à partir d'élément d'arrière-plan carrés de même taille, le deuxième type de plaques consiste en des plaques avec des symboles de test sur un arrière-plan formé à partir d'éléments d'arrière-plan circulaires gris et le troisième type de plaques consiste en des plaques avec des symboles de test sur un arrière-plan formé à partir d'éléments d'arrière-plan circulaires colorés.

2. Test selon la revendication 1, dans lequel lesdits éléments d'arrière-plan et lesdits symboles sont générés de manière aléatoire.

3. Test selon les revendications 1 à 2, dans lequel lesdits éléments et lesdits symboles de test ont trois niveaux distincts de luminosité, incluant lumineux, moyen et sombre.

4. Test selon les revendications 1 à 3, dans lequel lesdits symboles de test sur lesdits deuxième et troisième types de plaques sont choisis parmi un groupe de formes géométriques facilement identifiables, comprenant un cercle, un carré, un losange et une croix.

5. Test selon les revendications 1 à 4, dans lequel lesdits symboles de test sur le premier type de plaques sont un ou deux chiffres arabes.

6. Test selon les revendications 1 à 5, dans lequel lesdits premier et deuxième types de plaques contiennent quatre symboles de test.

7. Test selon les revendications 1 à 6, dans lequel ledit troisième type de plaques contient trois symboles de test.

8. Test selon les revendications 1 à 7, dans lequel ledit test contient 15 plaques qui sont adaptées pour la détermination de déficiences de la vision rouge-verte et deux plaques adaptées pour la détermination de la déficience de la vision de type bleu-jaune.

9. Test selon la revendication 8, dans lequel lesdites 15 plaques pour déterminer le type rouge-vert de déficiences de la vision sont divisées en cinq catégories, chaque catégorie comprenant au moins une plaque de chaque type, dans lequel lesdites cinq catégories sont conçues pour déterminer une déficience de la vision des couleurs minime, légère, moyenne, forte, et dichromatique respectivement.

10. Test selon la revendication 8, dans lequel lesdites deux plaques pour déterminer le type bleu-jaune de déficiences de la vision sont formées de cercles gris dans trois niveaux différents de luminosité et dans lequel chaque plaque de test contient quatre symboles de test avec chacun sa propre saturation de couleur, dans lequel les symboles de test sont des formes géométriques.

11. Test selon la revendication 10, dans lequel une desdites plaques de test comprend des symboles de test de teinte bleue.

12. Test selon les revendications 10 à 11, dans lequel une desdites plaques de test comprend des symboles de test de teinte jaune.

13. Procédé pour préparer un test de déficience de la vision des couleurs selon les revendications 1 à 12, ledit procédé comprenant le choix d'une réception de couleurs, l'acquisition de couches d'encre pour des valeurs graduées de CMJN, la création d'une table de consultation pour une imprimante particulière avec des paramètres particuliers, la fourniture d'une réception de CMJN théorique basée sur ladite table de consultation, pour tenir compte des lignes de confusion, de la luminosité et de la saturation de couleurs, l'impression d'échantillons de couleurs initiaux, l'analyse desdits échantillons de couleurs initiaux dans des conditions de couleurs réelles ; l'imagerie desdits échantillons de couleurs initiaux à l'aide d'une caméra multi-spectrale étalonnée, la comparaison desdites données colorimétriques acquises avec la réception de CMJN théorique, et l'impression des plaques de test à l'aide de la réception de CMJN corrigée.
